Europäisches Patentamt

European Patent Office

Office européen des brevets

(11) Publication number: **0 381 340**
**A2**

# (12) EUROPEAN PATENT APPLICATION

(21) Application number: **90300547.8**

(22) Date of filing: **18.01.90**

(51) Int. Cl.⁵: **C07K 7/06, C07K 7/30,**
**A61K 37/02**

(30) Priority: **26.01.89 US 303425**

(43) Date of publication of application:
**08.08.90 Bulletin 90/32**

(84) Designated Contracting States:
**GR**

(71) Applicant: **FISONS CORPORATION**
**Jefferson Road P.O. Box 1710**
**Rochester, New York 14603(US)**

(72) Inventor: **Rosamond, James D.**
**187 Burlington Avenue**
**Rochester, New York 14619(US)**

(74) Representative: **Wright, Robert Gordon McRae**
**et al**
**FISONS plc 12 Derby Road**
**Loughborough Leicestershire LE11 0BB(GB)**

(54) **Peptides with sulphate ester groups.**

(57) There are disclosed compounds of formula I:
Q - T - M - G - W - X - J - P - Z    I
wherein
Q represents H-Asp, H-βAsp, H-DAsp, For, Suc or $R^1R^2CHOCO$,
T represents $Tyr(SO_3H)$,
M represents Ile,
G represents Gly, D-Ala or Pro,
W represents Trp,
X represents Ahx,
J represents Asp,
P represents MePhe,
Z represents $NR^3R^4$,
$R^1$, $R^2$, $R^3$ and $R^4$ are independently H or lower alkyl,
in addition
    a) Q and T may together represent $Hpp(SO_3H)$,
    b) when Q represents H-βAsp, X may represent Ile,
    c) when G represents D-Ala, M and X may both represent Met
    d) when Q represents H-DAsp, then M and X may both represent Ile provided that P represents Phe,
    e) when Q represents H-Asp and G represents Pro, M and X may both represent Met. and
pharmaceutically acceptable salts thereof.
The compounds are useful for the treatment of obesity.

EP 0 381 340 A2

## Peptides with sulphate ester group (IR 2826C)

This invention relates to novel peptides with sulphate ester groups, pharmaceutical compositions containing them, methods for their preparation and the use of such compounds and compositions in the treatment of obesity.

Certain peptides are known which inhibit feeding, eg CCK-8, which has the structure Asp-Tyr(SO$_3$H)-Met-Gly-Trp-Met-Asp-Phe-NH$_2$ and ceruletide, which has the structure Glp-Gln-Asp-Tyr(SO$_3$H)-Thr-Gly-Trp-Met-Asp-Phe-NH$_2$. We have now found a group of novel peptides which have surprising and advantageous properties compared to known compounds of a similar structure.

According to the invention, there are provided compounds of formula I:

Q - T - M - G - W - X - J - P - Z     I

wherein

Q represents H-Asp, H-$\beta$Asp, H-DAsp, For, Suc or R$^1$R$^2$CHOCO,

T represents Tyr(SO$_3$H),

M represents Ile,

G represents Gly, D-Ala or Pro,

W represents Trp,

X represents Ahx,

J represents Asp,

P represents MePhe,

Z represents NR$^3$R$^4$,

R$^1$, R$^2$, R$^3$ and R$^4$ are independently H or lower alkyl,

in addition

    a) Q and T may together represent Hpp(SO$_3$H),

    b) when Q represents H-$\beta$Asp, X may represent Ile,

    c) when G represents D-Ala, M and X may both represent Met

    d) when Q represents H-DAsp, then M and X may also both represent Ile provided that P represents Phe,

    e) when Q represents H-Asp and G represents Pro, M and X may both represent Met, and pharmaceutically acceptable salts thereof.

According to the invention there is also provided a process for the production of compounds of formula I, or a pharmaceutically acceptable salt thereof, which comprises

a) sulphating a corresponding compound of formula II

Q$_p$ - T$_{OH}$ - M - G - W - X - J$_p$ - P - P$_c$     II

wherein Q$_p$ represents Q, P$_n$-HAsp, P$_n$-$\beta$Asp or P$_n$-DAsp, in which P$_n$ represents an amino protecting group, P$_c$ represents Z or a carboxyl protecting group,

T$_{OH}$ represents tyrosine,

J$_p$ represents J or a carboxyl protected aspartate and

M, G, W, X, J and P are as defined above,

with a sulphating reagent

and where desired or necessary removing any protecting groups from the resulting compound or

b) removing one or more protecting groups from a corresponding compound of formula III

Q$_p$ - T - M - G - W - X - J$_p$ - P - P$_c$     III

wherein Q$_p$, P$_c$, T, J$_p$, M, G, W, P and X are as defined above,

provided that at least one of Q$_p$, P$_c$ or J$_p$ represents or contains a protecting group and

where desired or necessary converting the resulting compound of formula I to a pharmaceutically acceptable salt thereof or vice versa.

In process a), the sulphating agent may be for example, sulphur trioxide or a complex thereof, such as sulphur trioxide pyridine. We particularly prefer to carry out the sulphation in a polar aprotic solvent, for example dimethylformamide or pyridine. The reaction is preferably carried out using an excess of sulphating agent, for example a 10 - 40 molar excess.

In process a) and b), protecting groups for peptides and methods for their removal are well known in the art, for example, T W Greene, Protective Groups in Organic Synthesis, Wiley-Interscience (1981). The choice of protecting groups and the methods employed for their removal will depend, inter alia, on the method of synthesis employed for the preparation of the peptide of formula III and the amino acids in the peptide. Suitable carboxyl protecting groups that P$_c$ may represent include, for example, methyl, tert-butyl, benzyl and 4-methoxybenzyl. We particularly prefer benzyl, which may be readily removed by treatment with alcoholic amine or ammonia. Similar groups may be used to protect the phenol group in tyrosine and the carboxyl group in aspartate.

When the peptide is prepared using solid phase techniques, eg those in which the carboxyl end of the peptide is attached to a solid phase resin, linkage of the peptide to the resin acts as a carboxyl protecting group. Cleavage of the peptidyl-resin linkage will deprotect the carboxyl terminus of the compounds of formula II. Since the sulphate ester containing peptide end products of this invention are carboxyl terminal amides, the chemical link which connects the peptide chain to the resin must be such that its cleavage with suitable reagents readily provides amides. Due to the lability of the sulphate ester group to strong acids (for example, liquid hydrogen fluoride), the peptidyl-resin linkage may be cleavable with either weaker acids (for example, brief treatment with trifluoroacetic acid, TFA) and/or nucleophiles (for example, ammonia, amines, hydroxide, and alkoxides).

Among the suitable resin derivatives may be mentioned oxymethyl-polystyrene, 4-(oxymethylphenyl)-$(CH_2)_n$-aminomethyl-polystyrene (n = 0-3) and 4-(oxymethylphenyl)oxymethyl-polystyrene. Similarly substituted polyacrylamide resins are equally well suited as the above polystyrene based resins. The term "polystyrene" includes copolymers with minor amounts, usually 1%, of unsaturated monomers such as divinylbenzene.

4-(Oxymethylphenyl)$CH_2$CO-aminomethyl-polystyrene [herein referred to as 4-(oxymethylphenyl)-acetamidomethyl-polystyrene or $OCH_2$-Pam-resin] is particularly preferred for the generation of peptide amides. This linkage may readily be cleaved to give the peptides of formula I by reaction with methanolic solutions of ammonia, alkylamines or dialkylamines as required.

Suitable amino protecting groups that $P_n$ may represent include, for example, benzyloxycarbonyl, which may readily be removed by hydrogenolysis or hydrogen bromide in acetic acid; t-butyloxycarbonyl, (Boc), which is removed by standing the peptide in cold trifluoroacetic acid; Fmoc, which may be removed by treatment with dilute piperidine (20% in DMF); (4-methoxybenzyl)oxycarbonyl and 2-nitrophenylsulphenyl. The Boc and Fmoc groups are particularly preferred.

Peptides of formula III may be prepared by sulphation of a corresponding protected peptide of formula II.

Unprotected peptides of formula II may be made by deprotection of a corresponding peptide of formula II.

The novel protected peptides of formula II and the novel intermediates thereof may be prepared by methods well known to the art, for example, they may be prepared by combining individual amino acids on a solid phase resin on a step-by-step basis, or alternatively, by combining groups of amino acids on a solid phase resin to yield the desired peptidyl-resin intermediate. Such additions, as is known, are accomplished by protecting the amino group of the amino acid or group of amino acids by converting it to, for example, its tert-butyloxycarbonyl (Boc) or 9-fluorenylmethyloxycarbonyl (Fmoc) derivative, and then activating the carboxylic group of such amino acid or group of amino acids by converting it, for example, to its 1-hydroxybenzotriazole (HOBt) or N-hydroxysuccinimide (HOSu) ester derivative. Such a protected-activated intermediate is then allowed to react with an amino acid resin or peptidyl-resin with a free amino group, thus extending the peptide chain to provide the peptidyl-resin of formula II, wherein $Q_p$ bears a suitable protecting group, for example, Boc or Fmoc.

The amino acid defined by the group P of formula II may be attached to the $OCH_2$-pam-resin in several ways. (a) For example, Boc-protected N-methylphenylalanine, may be reacted with a suitable 4-(bromomethyl)phenylacetate ester (for example, phenacyl ester) and processed further to provide Boc-MePhe(4-oxymethylphenyl)acetic acid which may be coupled to aminomethyl-polystyrene to provide Boc-MePhe-(4-oxymethylphenyl)acetamidomethylpolystyrene (Boc-MePhe-$OCH_2$-Pam-resin). (b) Alternatively, 4-(bromomethyl)phenylacetic acid may be coupled to aminomethylpolystyrene to provide 4-(bromo-methyl)phenylacetamidomethylpolystyrene (BrCH$_2$-Pam-resin) which may be reacted with the cesium salt of Boc-MePhe-OH to provide Boc-Phe-$OCH_2$-Pam-resin.

Among the suitable activating groups may be mentioned any combination of groups which causes the acid function of the amino acid to become more reactive, such as acid chlorides, mixed and symmetrical anhydrides, reaction product with carbodiimide (for example, dicyclohexylcarbodiimide, DCC), and active esters (for example, esters derived from HOBt, HOSu, 2- or 4-nitrophenol, and 2,4,5-trichlorophenol). The use of DCC and esters of HOBt and HOSu is particularly preferred from the standpoint of yield, lack of by-products, and consequent ease of purification.

An automatic peptide synthesizer was used for the solid phase synthesis of the sulfated peptide amides of this invention. The protocol of coupling onto aminomethylresin or peptidyl-$OCH_2$-Pam-resin (1 mmole of available nitrogen), deprotection, sulfation, cleavage, and product purification is set forth in Table 1.

Table 1. Protocol for solid phase synthesis of sulfated peptide amides (1 mmole scale).

Each step volume is 50 ml unless otherwise indicated. All wash steps are repeated three times. Abbreviations: DCC, dicyclohexylcarbodiimide; DCM, dichloromethane; DIEA, N,N-diisopropylethylamine, DMF, dimethylformamide; HOBt, 1-hydroxybenzotriazole; TFA, trifluoroacetic acid.

## TABLE 1

| Step | Reagent or Solvent | Purpose | Mix Time |
|---|---|---|---|
| 1 | DCM | Wash | 1 min |
| 2 | Go to Step 3, 5, or 8 | - | - |
| 3 | Add filtered, pre-activated (0°C, 1 hr) mixture of protected amino acid (or protected dipeptide, HOBt (4.5 mmole), and DCC (3 mmole), in 1:4 DMF/DCM | Pre-activated DCC/HOBt coupling | 2-15 hr |
| 4 | Go to step 10, 16, 21 or 26 | - | - |
| 5 | Add protected amino acid (or protected dipeptide, 3 mmole) and HOBt (4.5 mmole) in 30ml 1:2 DMF/DCM then DCC (3 mmole) in 20 ml DCM | In situ activated DCC/HOBt coupling | 2-15 hr |
| 6 | 2-Propanol | Wash | 1 min |
| 7 | Go to step 4 | - | - |
| 8 | Add active ester or anhydride (3 mmole) in DCM, DMF, or mixture thereof | Non DCC/HOBt activated coupling | 2-15 hr |
| 9 | Go to Step 4 | - | - |
| 10 | DCM | Wash | 1 min |
| 11 | Treat with 49:1:50 TFA/anisole/DCM | BOC and tBu removal | 30 min |
| 12 | DCM | Wash | 1 min |
| 13 | Treat with 1:19 DIEA/DCM | Neutralise | 1 min |
| 14 | DCM | Wash | 1 min |
| 15 | Go to step 1, 16, 21 or 26 | - | - |
| 16 | DMF | Wash | 1 min |
| 17 | Treat with 1:4 piperidine/DMF | Fmo6 removal | 3 min |
| 18 | Treat with 1:4 piperidine/DMF | Fmoc removal | 7 min |
| 19 | DMF | Wash | 1 min |
| 20 | Go to Step 15 | - | - |
| 21 | DMF | Wash | 1 min |
| 22 | 1:2 pyridine/DMF | Wash | 1 min |
| 23 | Add sulfur trioxide pyridine complex (40 mmole) in 60ml 1:2 pyridine/DMF | Sulfation | 20-24 hr |
| 24 | DMF | Wash | 1 min |
| 25 | Go to Step 4 | - | - |
| 26 | Methanol | Wash | 1 min |
| 27 | Ammonia saturated (-20°C) methanol or 20% methanolic amine (250ml) | Resin cleavage | 2-5 day |
| 28 | Methanol | Wash | 1 min |

| Step Reagent or Solvent | Purpose | Mix Time |
|---|---|---|
| 29 Combine, concentrate filtrates from steps 27-28 | Isolation | - |
| 30 Chromatograph residue on column(s) of Amberlite XAD-2 (Rohm and Haas, 2.5x60cm, methanol gradient 0.1M in ammonia), Trisacryl M MEAE (LKB Inc., 2.5x47cm, ammonium bicarbonate gradient), and/or P-40 ODS-3 (Whatman, 4.8x50cm, methanol gradient 0.2% in ammonium acetate) | Purification | - |

The sulfate ester containing peptides of formula (1) thus prepared may be desalted and purified by the usual methods. For example, the product may be purified by ion-exchange chromatography with use of Trisacryl M DEAE, DEAE-cellulose or the like, partition chromatography with use of Sephadex LH-20, Sephadex G-25 or the like, reverse phase chromatography with use of Amberlite XAD-2, ODS-silica gel or the like, normal phase chromatography with use of silica gel or the like, or high-performance liquid chromatography (HPLC).

Analogous procedures, wherein the reactions are carried out without the solid phase component (resin), are well known in the art and well suited to large scale production. [See e.g., U.S. Patent 3,892,726].

Pharmaceutically acceptable salts of the compounds of formula I that may be mentioned include pharmaceutically acceptable base salts. Base salts include those derived from both organic and inorganic bases, such as, for example, ammonia, sodium hydroxide, calcium hydroxide, barium hydroxide, tetraethylammonium hydroxide, ethylamine, diethylamine, triethylamine, and the like.

When one or more of $R^1$, $R^2$, $R^3$ and $R^4$ represent alkyl, they preferably contain up to six, more preferably up to four carbon atoms. Particular groups that may be mentioned include methyl and ethyl. A particular group that $R^1R^2CHOCO$ may represent is iBuOCO.

We prefer compounds of formula I in which G represents Gly.

We prefer compounds of formula I in which Z represents $NH_2$.

We prefer compounds of formula I in which Q represents $\beta$Asp, For and particularly Suc.

A particularly preferred compound of formula I is Suc-Tyr($SO_3H$)-Ile-Gly-Trp-Ahx-Asp-MePhe-$NH_2$.

The peptides of Formula I, and the pharmaceutically acceptable salts therof, are useful because they possess pharmacological activity in animals; in particular they are useful because they have the ability to inhibit feeding activity in mammals. As a result they have utility in the prevention and treatment of obesity. Feeding inhibition activity can be demonstrated in rats as follows:

Male Sprague-Dawley rats (weighing 300-350 g) are individually caged and maintained on a 12 hr. light, dark cycle and trained for at least 14 days to feed during a three hr period of the dark cycle but not the 21 hours preceding that three hr period. The day of the study, rats are dosed intraperitoneally with saline (controls) or test compound (dissolved in saline; usually at a concentration of 0.3 to 300 micrograms of test compound per kg of rat weight). Food is introduced 10 minutes after administration of saline or test compound. Test compounds are deemed to be active if the test group consumes significantly less food than the saline controls during the feeding period, which ends either 0.5 or 3 hr after presentation of food.

The peptides of this invention have the ability to stimulate gallbladder contraction in mammals. Thus, they also find utility as diagnostic aids in X-ray examination of the gallbladder. The use of gallbladder contracting agents as diagnostic aids is a well established medical procedure.

The compounds of formula I, and pharmaceutically acceptable salts thereof, have the advantage that in certain pharmacological models, they are more efficacious, more potent, longer acting, more stable, particularly to enzymatic degradation, more selective, less toxic, give rise to fewer side effects, eg lack of emesis, are more readily absorbed, are quicker acting or have other advatageous effects compared to compounds of similar structure to the compounds of formula I.

According to the invention there is also provided the use of the compounds of formula I, and pharmaceutically acceptablesalts thereof, in the manufacture of a medicament for use in the treatment of obesity.

According to the invention we also provide a pharmaceutical composition comprising (preferably less than 80%, and more preferably less than 50% by weight of) a compound of formula I, or a pharmaceutically acceptable salt thereof, in combination with a pharmaceutically acceptable adjuvant, diluant or carrier.

The compositions may be designed for intraperitoneal, intravenous, intramuscular, subcutaneous, or intranasal administration.

According to the invention, there is also provided a method of treatment of obesity, which comprises administering an effective amount of a compound of formula I, or a pharmaceutically acceptable salt thereof,to a mammal in need of either treatment for obesity or prevention of obesity. An amount of the compound of formula I that may be mentioned is a dose of about 0.03g to 3 mg per kg of body weight per day, either as single dose or divided among two to four doses.

The dosage may be varied, depending upon the requirements of the patient and the compound being employed.

In an alternative representation to formula (I), used for purposes of brevity, peptides are also represented in accordance with conventional representation, for example,

Suc-Tyr(SO$_3$H)-Ile-Gly-Trp-Ahx-Asp-MePhe-NH$_2$, which stands for the compound of formula (1) in which Q is Suc, T is Tyr(SO$_3$H), M is Ile, G is Gly, W is Trp, X is Ahx, J is Asp, P is MePhe and Z is NH$_2$.

All optically active amino acids are of the L-configuration unless otherwise indicated.

When amino acids, peptides, protecting groups, active groups, etc. are represented by symbols in this specification and appended claims, usual symbols as defined by IUPAC and IUB or as used in the art are employed.

Examples of symbols are given below.

Ahx.................2-aminohexanoic acid
Ala.................alanine
Arg.................arginine
Asn.................asparagine
Asp.................aspartic acid
$\beta$Asp...............beta-aspartic acid
Boc..................tert-butyloxycarbonyl
DAla...............D-alanine
Fmoc..............9-fluorenylmethyloxycarbonyl
For.................formyl
Gln.................glutamine
Glp.................pyroglutamyl
Gly.................glycine
Hpp.................3-(4-hydroxyphenyl)propionyl
Hpp(SO$_3$H).....3-(0-sulfo-4-oxyphenyl)propionyl
iBuOCO..............isobutyloxycarbonyl
Ile....................isoleucine
Leu...................leucine
Lys...................lysine
MePhe..............N-methylphenylalanine
MePhe-NH:........N-methylphenylalanine amide
Met.................methionine
OBt..................1-benzotriazolyl ester
OCH$_2$-Pam.........4-(oxymethylphenyl)acetamidomethyl
OSu.................succinimidyloxy ester
OtBu................tert-butyl ester
Phe..................phenylalanine
Pro...................proline
resin................polystyrene
Suc...................HOOC(CH$_2$)$_2$CO-
tBu...................tert-butyl
Thr...................threonine
Trp...................tryptophan
Tyr...................tyrosine
Tyr-NH:.............tyrosine amide
Tyr(SO$_3$H)...........O-sulfotyrosine
Tyr(SO$_3$H)-NH$_2$...O-sulfotyrosine amide

EXAMPLES

The invention may be further illustrated by the following examples.

Peptide syntheses, unless otherwise stated, were initiated with 1 milliequivalent of aminomethyl resin, where the resin was 99:1 by weight styrene:divinylbenzene copolymer. Reactions were performed at room temperature unless otherwise stated. Washing steps were performed three times with 50 ml of the specified solvent unless otherwise stated.

1. Preparation of Intermediates

Intermediate A

Boc-MePhe-(4-oxymethylphenyl)acetic Acid

To a solution of Boc-MePhe-OH (27.93 g) and 4-(bromomethyl) phenylacetic acid phenacyl ester (33.32 g) in 1000 ml of acetonitrile was added potassium fluoride dihydrate (18.28 g). The suspension was stirred overnight, filtered and the filtrate evaporated to dryness. The residue, Boc-MePhe-(4-oxymethylphenyl)-acetic acid phenacyl ester, was dissolved in 85% acetic acid (1200 ml), treated with zinc dust (128 g), and stirred for 2-4 hrs. Concentration of the filtered reaction mixture to ca. 400 ml and dilution with ca. 3200 ml of water gave an oil which was dissolved in EtOAC and treated with dicyclohexylamine (DCHA) to give 41.31 g of the DCHA salt of title compound, mp 120-122° C.

Intermediate B

Fmoc-Met-Asp(OtBu)-OH

Fmoc-Met-OSu was prepared, in situ, by the reaction of Fmoc-Met-OH (14.87 g) HOSu (5.52 g), and dicyclohexyl carbodiimide (DCC, 8.26 g) in tetrohydrofuran (THF, 200 ml) at 0° C for 3.5 hrs. Precipitated dicyclohexylurea (DCU) was removed by filtration and the THF filtrate was added to a cold solution of H-Asp (OtBu)-OH in 220 ml of 10:1 water/THF to which had been added 40 ml of $\underline{N}$ sodium hydroxide. After stirring the reaction mixture at room temperature overnight, solid citric acid (20 g) was added along with EtOAc (600 ml). The EtOAc layer was separated, washed with 10% citric acid and brine, and dried (magnesium sulphate). Evaporation of the EtOAc solution gave a residue which was dissolved in 200 ml of EtOAc and treated with DCHA (7.84 ml) to precipitate 17.93 g of the DCHA salt of the desired product, mp 159-162° C.

Intermediate C

H-PHe-OCH$_2$-Pam-resin

Boc-Phe-(4-oxymethylphenyl) acetic acid (0.83 g, 2 mmole), 1-hydroxybenzotriazole (HOBt, 0.46 g, 3 mmole) and DCC (0.41 g, 2 mmole) were dissolved in 50 ml of 4:1 DCM/DMF and stirred at 0° C for 1 hour. Aminomethyl-resin (1.34 g, 1 mmole available nitrogen (was suspended in the filtered reaction mixture (precipitated DCU removed) and shaken for 2 to 15 hours. The product, Boc-Phe-OCH$_2$-Pam-resin, was isolated by filtration and treated according to Table 1 (Steps 10-14) to give the desired base, H-Phe-OCH$_2$-Pam-resin.

Intermediate D

M-MePhe-OCH$_2$-Pam-resin

Boc-MePhe-(4-oxymethylphenyl) acetic acid (from 1.82 g, 3 mmole, of its DCHA salt, intermediate A) and HOBt (0.69 g, 4.5 mmole) in 40 ml of 1:3 DMF/DCM followed by DCC (0.62 g, 3 mmole) in 20 ml of DCM were added to aminomethyl-resin (1.34 g, 1 mmole available nitrogen) to give a suspension which was shaken for 2 to 15 hours. The desired product, Boc-MePhe-OCH$_2$-Pam-resin, was isolated by filtration, washed with 2-propanol and DCM, and treated according to Table 1 (Steps 10-14) to give the desired free base, H-MePhe-OCH$_2$-Pam-resin.

Intermediate $\underline{E}$

Isobutyl succinimidyl carbonate (iBuoco-OSu)

To a solution of isobutyl chloroformate (26 ml, 200 mmole) in 600 ml of chloroform was added in portions the dicyclohexylamine salt of N-hydroxysuccinimide (HOSu, 49.28g, 200 mmole). After stirring the resulting suspension overnight, the precipitated DCHA hydrochloride was filtered off and washed with chloroform. The concentrated filtrate (ca. 50 ml) and washings were diluted with 400 ml of ethyl acetate (EtOAc) and washed with 10% citric acid (4 x 100 ml), brine (2 x 100 ml), 10% sodium bicarbonate (3 x 100 ml), and brine (4 x 100ml) and then dried (magnesium sulphate), filtered, and concentrated to ca. 100 ml. On diluting with ether and precipitating with hexane, 26.6g (62% yield) of iBuOCO-OSu was obtained, mp 33-35° C.

2. Preparation of Examples of Compounds of Formula I

EXAMPLE 1

H-DAsp-Tyr(SO$_3$H)-Ile-Gly-Trp-Ile-Asp-Phe-NH$_2$

H-Phe-OCH$_2$-Pam-resin (Intermediate C) was sequentially coupled with Fmoc-Ile-Asp(OtBu)-OH (Intermediate B, Fmoc-Met-OH replaced with Fmoc-Ile-OH), Fmoc-Trp-OH, Fmoc-Gly-OH, Fmoc-Ile-OH, Fmoc-Tyr(tBu)-OH and Fmoc-DAsp(OtBu)-OH according to Table 1 (coupling Steps 5-7 followed by Fmoc removal Steps 16-20) to provide Fmoc-DAsp(OtBu)-Tyr(tBu)-Ile-Gly-Trp-Ile-Asp(OtBu)-Phe-OCH$_2$-Pam-resin which was deprotected, sulphated, deprotected, and cleaved from the resin according to Table 1 (Steps 10-15, Steps 21-25, Steps 16-20, and then Steps 26-29 with ammonia) to give the title compound which was chromatographically purified on Amberlite XAD-2 and P-40 ODS-3, sequentially, according to Table 1 (Step 30) to give 270 mg of the ammonium salt of the title compound. Amino acid analysis following acid decomposition gave Tyr 1.00 (1), Ile 1.94 (2), Gly 0.98 (1), Asp 2.14 (2), and Phe 0.94 (1). Infrared absorption spectrum showed a strong peak typical of a sulfuric acid ester at 1050 cm$^{-1}$. TLC R$_f$ 0.41.

EXAMPLE 2

H-Asp-Tyr(SO$_3$H)-Met-Pro-Trp-Met-Asp-MePhe-NH$_2$

H-MePhe-OCH$_2$-Pam-resin (Intermediate D) was sequentially coupled with Fmoc-Met-Asp(OtBu)-OH (Intermediate B), Fmoc-Trp-OH, Fmoc-Pro-OH, Fmoc-Met-OH, Fmoc-Tyr-OH, and Boc-Asp(OtBu)-OH according to Table 1 (coupling Steps 3-4 followed by Fmoc removal Steps 16-20) to provide Boc-Asp(OtBu)-Tyr-Met-Pro-Trp-Met-Asp(OtBu)-MePhe-OCH$_2$-Pam-resin which was sulfated, deprotected, and cleaved from the resin according to Table 1 (Steps 21-25, Steps 10-15, and then Steps 26-29 with ammonia) to give the title compound which was chromatographically purified on Trisacryl M DEAE and P-40 ODS-3, sequentially, according to Table 1 (Step 30) to give 83 mg of the ammonium salt of the title compound. Amino acid analysis following acid decomposition gave Asp 1.99 (2), Tyr 1.00 (1), Met 1.91 (2), and Pro

8

1.11 (1). Infrared absorption spectrum showed a strong peak typical of a sulphuric acid ester at 1050 cm$^{-1}$. TLC Rf 0.34.

## EXAMPLE 3

iBuOCO-Tyr(SO$_3$H)-Met-DAla-Trp-Met-Asp-MePhe-NH$_2$

H-MePhe-OCH$_2$-Pam-resin (Intermediate D) was sequentially coupled with Fmoc-Met-Asp(OtBu)-OH (Example B), Fmoc-Trp-OH, Fmoc-DAla-OH, Fmoc-Met-OH, and Fmoc-Tyr(tBu)-OH according to Table 1 (coupling Steps 5-7 followed by Fmoc removal Steps 16-20) to provide H-Tyr(tBu)-Met-DAla-Trp-Met-Asp-(OtBu)-MePhe-OCH$_2$-Pam-resin which was coupled with iBuOCOOSu (Intermediate E) in DMF according to Table 1 (Steps 8-9) to give iBuOCO-Tyr(tBu)-Met-DAla-Trp-Met-Asp(OtBu)-MePhe-OCH$_2$Pam-resin which was deprotected, sulfated, and cleaved from the resin according to Table 1 (Steps 10-15, Steps 21-25, and then Steps 26-29 with ammonia) to give the title compound which was chromatographically purified on Amberlite XAD-2 and P-40 ODS-3, sequentially, according to Table 1 (Step 30) to give 80 mg of the ammonium salt of the title compound. Amino acid analysis following acid decomposition gave Tyr 1.02 (1), Met 1.84 (2), Ala 1.05 (1), Asp 1.05 (1), and MePhe 1.04 (1). Infrared absorption spectrum showed a strong peak typical of a sulphuric acid ester at 1050 cm$^{-1}$. TLC R$_f$ 0.95.

## EXAMPLE 4

H-βAsp-Tyr(SO$_3$H)-Ile-Gly-Trp-Ile-Asp-MePhe-NH$_2$

H-MePhe-OCH$_2$-Pam-resin (Intermediate D) was sequentially coupled with Fmoc-Ile-Asp(OtBu)-OH (Intermediate B, Fmoc-Met-OH replaced with Fmoc-Ile-OH), Fmoc-Trp-OH, Fmoc-Gly-OH, Fmoc-Ile-OH, Fmoc-Tyr(tBu)-OH and Boc-βAsp(OtBu)-OH according to Table 1 (coupling Steps 5-7 followed by Fmoc removal Steps 16-20) to provide Boc-βAsp(OtBu)-Tyr(tBu)-Ile-Gly-Trp-Ile-Asp(OtBu)-MePhe-OCH$_2$-Pam-resin which was deprotected according to Table 1 (Steps 10-15) and coupled with Fmoc-OSu according to Table 1 (Steps 8-9) to give Fmoc-βAsp-Tyr-Ile-Gly-Trp-Ile-Asp-MePhe-OCH$_2$-Pam-resin which was sulphated, deprotected and cleaved from the resin according to Table 1 (Steps 21-25, Steps 16-20, and then Steps 26-29 with ammonia) to give the title compound which was chromatographically purified on Amberlite XAD-2 and P-40 ODS-3, sequentially, according to Table 1 (Step 30) to give 100 mg of the ammonium salt of the title compound. Amino acid analysis following acid decomposition gave Asp 2.01 (2), Tyr 1.00 (1), Ile 1.98 (2), Gly 1.02 (1), and MePhe 0.95 (1). Infrared absorption spectrum showed a strong peak typical of a sulphuric acid ester at 1050 cm$^{-1}$. TLC R$_f$ 0.47.

## EXAMPLE 5

For-Tyr(SO$_3$H)-Ile-Gly-Trp-Ahx-Asp-MePhe-NH$_2$

H-MePhe-OCH$_2$-Pam-resin (Intermediate D) was sequentially coupled with Fmoc-Ahx-Asp(OtBu)-OH (Intermediate B, Fmoc-Met-OH replaced with Fmoc-Ahx-OH), Fmoc-Trp-OH, Fmoc-Gly-OH, and Fmoc-Ile-OH according to Table 1 (coupling Steps 5-7 followed by Fmoc removal Steps 16-20) to provide H-Ile-Gly-Trp-Ahx-Asp(OtBu)-MePhe-OCH$_2$-Pam-resin which was deprotected according to Table 1 (Steps 10-15) to provide H-Ile-Gly-Trp-Ahx-Asp-MePhe-OCH$_2$-Pam-resin which was coupled with For-Tyr-OH according to Table 1 (Steps 3-4) to give For-Tyr-Ile-Gly-Trp-Ahx-Asp-MePhe-OCH$_2$-Pam-resin which was sulphated and cleaved from the resin according to Table 1 (Steps 21-25 and then Steps 26-29 with ammonia) to give the title compound which was chromatographically purified on Amberlite XAD-2 and P-40 ODS-3, sequentially, according to Table 1 (Step 30) to give 60 mg of the ammonium salt of the title compound. Amino acid analysis following acid decomposition gave Tyr 0.99 (1), Ile 1.01 (1), Ahx 1.05 (1), Gly 1.04 (1), Asp 1.05 (1), and MePhe 0.86 (1). Infrared absorption spectrum showed a strong peak typical of a sulphuric acid ester at 1050 cm$^{-1}$. TLC Rf 0.51.

EXAMPLE 6

Suc-Tyr(SO$_3$H)-Ile-Gly-Trp-Ahx-Asp-MePhe-NH$_2$

H-MePhe-OCH$_2$-Pam-resin (Intermediate D) was sequentially coupled with Fmoc-Ahx-Asp(OtBu)-OH (Intermediate B, Fmoc-Met-OH replaced with Fmoc-Ahx-OH), Fmoc-Trp-OH, Fmoc-Gly-OH, Fmoc-Ile-OH, and Fmoc-Tyr(tBu)-OH according to Table 1 (coupling Steps 5-7 followed by Fmoc removal Steps 16-20) to provide H-Tyr(tBu)-Ile-Gly-Trp-Ahx-Asp(OtBu)-MePhe-OCH$_2$-Pam-resin which was coupled with succinic anhydride in DMF according to Table 1 (Steps 8-9) to give Suc-Tyr(tBu)-Ile-Gly-Trp-Ahx-Asp(OtBu)-MePhe-OCH$_2$-Pam- resin which was deprotected, sulphated, and cleaved from the resin according to Table 1 (Steps 10-15, Steps 21-25, and then Steps 26-29 with ammonia) to give the title compound which was chromatographically purified on Amberlite XAD-2 and P-40 ODS-3, sequentially, according to Table 1 (Step 30) to give 130 mg of the ammonium salt of the title compound. Amino acid analysis following acid decomposition gave Tyr 0.99 (1), Ile 0.94 (1), Ahx 0.96 (1), Gly 1.02 (1), Asp 1.00 (1), and MePhe 1.09 (1). Infrared absorption spectrum showed a strong peak typical of a sulphuric acid ester at 1050 cm$^{-1}$. TLC R$_f$ 0.65.

EXAMPLE 7

iBuOCO-Tyr(SO$_3$H)-Ile-Gly-Trp-Ahx-Asp-MePhe-NH$_2$

H-MePhe-OCH$_2$-Pam-resin (Intermediate D) was sequentially coupled with Fmoc-Ahx-Asp(OtBu)-OH (Intermediate B, Fmoc-Met-OH replaced with Fmoc-Ahx-OH), Fmoc-Trp-OH, Fmoc-Gly-OH, Fmoc-Ile-OH, and Fmoc-Tyr(tBu)-OH according to Table 1 (coupling Steps 5-7 followed by Fmoc removal Steps 16-20) to provide H-Tyr(tBu)-Ile-Gly-Trp-Ahx-Asp(OtBu)-MePhe-OCH$_2$-Pam-resin which was coupled with iBuOCO-OSu (Intermediate E) in DMF according to Table 1 (Steps 8-9) to give iBuOCO-Tyr(tBu)Ile-Gly-Trp-Ahx-Asp-(OtBu)-MePhe-OCH$_2$-Pam-resin which was deprotected, sulphated, and cleaved from the resin according to Table 1 (Steps 10-15, Steps 21-25, and then Steps 26-29 with ammonia) to give the title compound which was chromatographically purified on Amberlite XAD-2 and P-40 ODS-3, sequentially, according to Table 1 (Step 30) to give 140 mg of the ammonium salt of the title compound. Amino acid analysis following acid decomposition gave Tyr 1.05 (1), Ile 1.04 (1), Ahx 1.03 (1), Gly 1.06 (1), Asp 1.07 (1), and MePhe 0.76 (1). Infrared absorption spectrum showed a strong peak typical of a sulphuric acid ester at 1050 cm$^{-1}$. TLC R$_f$ 0.93.

EXAMPLE 8

Hpp(SO$_3$H)-Ile-Gly-Trp-Ahx-Asp-MePhe-NH$_2$

H-MePhe-OCH$_2$-Pam-resin (Intermediate D) was sequentially coupled with Fmoc-Ahx-Asp(OtBu)-OH (Intermediate B, Fmoc-Met-OH replaced with Fmoc-Ahx-OH), Fmoc-Trp-OH, Fmoc-Gly-OH, and Fmoc-Ile-OH according to Table 1 (coupling Steps 5-7 followed by Fmoc removal Steps 16-20) to provide H-Ile-Gly-Trp-Ahx-Asp(OtBu)-MePhe-OCH$_2$-Pam-resin which was coupled with Hpp-OSu according to Table 1 (coupling Steps 8-9) to give Hpp-Ile-Gly-Trp-Ahx-Asp(OtBu)-MePhe-OCH$_2$-Pam-resin which was deprotected, sulphated, and cleaved from the resin according to Table 1 (Steps 10-15, Steps 21-25, and then Steps 26-29 with ammonia) to give the title compound which was chromatographically purified on Amberlite XAD-2 and P40 ODS-3, sequentially, according to Table 1 (Step 30) to give 30 mg of the ammonium salt of the title compound. Amino acid analysis following acid decomposition gave Ile 0.86 (1), Ahx 0.82 (1), Gly 1.16 (1), and Asp 1.17 (1). Infrared absorption spectrum showed a strong peak typical of a sulphuric acid ester at 1050 cm$^{-1}$. TLC R$_f$ 0.64.

**Claims**

1. A process for the preparation of compounds of formula I:

Q - T - M - G - W - X - J - P - Z     I

wherein

Q represents H-Asp, H-$\beta$Asp, H-DAsp, For, Suc or $R^1R^2$CHOCO,

T represents Tyr($SO_3H$),

M represents Ile,

G represents Gly, D-Ala or Pro,

W represents Trp,

X represents Ahx,

J represents Asp,

P represents MePhe,

Z represents $NR^3R^4$,

$R^1$, $R^2$, $R^3$ and $R^4$ are independently H or lower alkyl,

in addition

a) Q and T may together represent Hpp($SO_3H$),

b) when Q represents H-$\beta$Asp, X may represent Ile,

c) when G represents D-Ala, M and X may both represent Met

d) when Q represents H-DAsp, then M and X may both represent Ile provided that P represents Phe,

e) when Q represents H-Asp and G represents Pro, M and X may both represent Met.

and pharmaceutically acceptable salts thereof, which comprises

a) sulphating a corresponding compound of formula II

$Q_p$ - $T_{OH}$ - M - G - W - X - $J_p$ - P - $P_c$     II

wherein $Q_p$ represents Q, $P_n$-H-Asp, $P_n$-$\beta$Asp or $P_n$-DAsp, in which $P_n$ represents an amino protecting group,

$P_c$ represents Z or a carboxyl protecting group,

$T_{OH}$ represents tyrosine,

$J_p$ represents J or a carboxyl protected aspartate and

M, G, W, X, J and P are as in claim 1,

with a sulphating reagent

and where desired or necessary removing any protecting groups from the resulting compound or

b) removing one or more protecting groups from a corresponding compound of formula III

$Q_p$ - T - M - G - W - X - $J_p$ - P - $P_c$     III

wherein $Q_p$, $P_c$, T, $J_p$, M, G, W, P and X are as defined above,

provided that at least one of $Q_p$, $P_c$ or $J_p$ represents or contains a protecting group and

and where desired or necessary converting the resulting compound of formula I to a pharmaceutically acceptable salt or vice versa.

2. A process according to claim 1, wherein G represents Gly.

3. A process according to claim 1 or claim 2, wherein Z represents $NH_2$.

4. A process according to any one of the preceding claims, wherein Q represents $\beta$Asp, For or Suc.

5. A process according to any one of the preceding claims, wherein the compound of formula I is Suc-Tyr($SO_3H$)-Ile-Gly-Trp-Ahx-Asp-MePhe-$NH_2$, or a pharmaceutically acceptable salt thereof.

6. A process according to any one of claims 1 to 5, wherein the compound of formula I is H-DAsp-Tyr-($SO_3H$)-Ile-Gly-Trp-Ile-Asp-Phe-$NH_2$, H-Asp-Tyr($SO_3H$)-Met-Pro-Trp-Met-Asp-MePhe-$NH_2$, iBuOCO-Tyr-($SO_3H$)-Met-DAla-Trp-Met-Asp-MePhe-$NH_2$, H-$\beta$Asp-Tyr($SO_3H$)-Ile-Gly-Trp-Ile-Asp-MePhe-$NH_2$, For-Tyr-($SO_3H$)-Ile-Gly-Trp-Ahx-Asp-MePhe-$NH_2$, iBuOCO-Tyr($SO_3H$)-Ile-Gly-Trp-Ahx-Asp-MePhe-$NH_2$, Hpp($SO_3H$)-Ile-Gly-Trp-Ahx-Asp-MePhe-$NH_2$

or a pharmaceutically acceptable salt of any one thereof.

7. A process for the preparation of a pharmaceutical composition which comprises a mixing a compound of formula I according to any one of the preceding claims with a pharmaceutically acceptable adjuvant, diluent or carrier.